# EUROPÄISCHE PATENTSCHRIFT

(11) **EP 0 634 397 B1**
(45) Veröffentlichungstag und Bekanntmachung des Hinweises auf die Patenterteilung: **09.10.1996**
(21) Anmeldenummer: 94110442.4
(22) Anmeldetag: 05.07.1994
(51) Int. Cl.: C07D 209/42

(54) **Verfahren zur Herstellung eines Indolderivates**
Process for the preparation of indole derivatives
Procédé pour la préparation des dérivés d'indole

(30) Priorität: 17.07.1993 DE 4324043
(43) Veröffentlichungstag der Anmeldung: 18.01.1995
(73) Patentinhaber: MERCK PATENT GmbH, D-64293 Darmstadt (DE)
(72) Erfinder: Hopf, Martin, Dr., D-64846 Gross-Zimmern (DE)

(56) Entgegenhaltungen:
- JOURNAL OF THE CHEMICAL SOCIETY, PERKIN TRANSACTIONS 2, Bd.8, 1982, LETCHWORTH GB Seiten 909 - 916 D.W. CLACK ET AL. 'Electrophilic substitution in indoles.'
- CHEMICAL AND PHARMACEUTICAL BULLETIN, Bd.25, Nr.11, 1977, TOKYO JP Seiten 3023 - 3033 T.NAGASAKA AND S. OHKI 'Indoles.'

## Beschreibung

Die Erfindung betrifft ein neues Verfahren zur Herstellung von 3-(3-Carboxypropyl)-5-methoxyindol-2-carbonsäure (I).

Diese Verbindung wurde bisher hergestellt, indem man 6-Cwbethoxy-6-p-methoxyphenylhydrazono-hexansäure (II) mit Schwefelsäure/Ethanol zu I-Diethylester umsetzte (gleichzeitige Veresterung!), diesen isolierte und anschließend mit ethanolischer Natronlauge verseifte (verbesserte Ausführungsform des in J. Chem. Soc. Perkin Trans. II 1982, 909-916 beschriebenen Verfahrens). Die Gesamtausbeute bei diesem Verfahren (54 %) befriedigte jedoch nicht.

Auch Versuche, höhere Ausbeuten zu erzielen, indem die Cyclisierung des Hydrazons II in Gegenwart einer Lewissäure in Essigsäure durchgeführt wurde, wie es für eine ähnliche Cyclisierung von Nagasaka und Ohki (Chem. Pharm. Bull. 25 (11) 3034-3033 (1977) beschrieben wurde, führten nicht zu dem gewünschten Erfolg.

Der Erfindung lag die Aufgabe zugrunde, ein verbessertes Verfahren zur Herstellung von I aufzufinden.

Es wurde gefunden, daß man den Ringschluß von II auch mit einer Carbonsäure, die 1-3 C-Atome enthält, durchführen kann. Allerdings erhält man dabei ein Gemisch der Dicarbonsäure I mit ihren Mono- und Diethylestern. Dieses Gemisch kann jedoch direkt mit einer starken anorganischen Base behandelt und nachfolgend angesäuert werden, wobei in guter Ausbeute I erhalten wird.

Gegenstand der Erfindung ist ein Verfahren zur Herstellung von 3-(3-Carboxypropyl)-5-methoxyindol-2-carbonsäure (I), dadurch gekennzeichnet, daß man 6-Carbethoxy-6-p-methoxyphenylhydrazonohexansäure (II) mit einer Carbonsäure, die 1-3 C-Atome enthält, cyclisiert, das erhaltene Gemisch aus I, den beiden I-Monoethylestern und I-Diethylester mit einer starken anorganischen Base behandelt und das erhaltene Salz durch Behandeln mit einer starken Säure in die freie Säure I umwandelt.

Die Säure I kann als Zwischenprodukt bei der Herstellung von Roxindol verwendet werden.

Die Cyclisierung von II mit der Carbonsäure wird zweckmäßig bei Temperaturen zwischen 20 und 120° durchgeführt, vorzugsweise bei Siedetemperatur. Die Reaktionszeiten liegen zwischen etwa 1 und 200 Stunden, vorzugsweise zwischen 10 und 30 Stunden. Man verwendet zweckmäßig etwa 0,5 bis 10, vorzugsweise 1 bis 2 Gewichtsteile Carbonsäure, bezogen auf II. Die Carbonsäure wird vorzugsweise in wasserfreier Form verwendet. Ein geringer Wassergehalt (bis ca. 10 %) verringert die Ausbeute um einige Prozente, ist aber sonst nicht schädlich. Höhere Wassergehalte führen zu schlechteren Ausbeuten. Als Carbonsäure ist Essigsäure bevorzugt, aber auch Ameisensäure oder Propionsäure sind gut geeignet.

Zweckmäßig dampft man das Reaktionsgemisch anschließend ein und behandelt das erhaltene Gemisch mit einer starken anorganischen Base, vorzugsweise einem Alkalimetallhydroxid, insbesondere KOH, aber auch z.B. NaOH, oder einem Erdalkalimetallhydroxid, z.B. Ca(OH)₂, in einem inerten Lösungsmittel oder Lösungsmittelgemisch, z.B. einem Alkohol wie Methanol, Ethanol oder Isopropanol oder Wasser oder einem Alkohol/Wasser-Gemisch, bei Temperaturen zwischen etwa 20 und 120°, vorzugsweise zwischen 60 und 100°.

Schließlich säuert man an, zweckmäßig mit einer starken Säure, z.B. einer Mineralsäure wie Salzsäure oder Schwefelsäure, worauf die freie Säure I ausfällt. Vorteilhaft neutralisiert man zunächst, klärt die Lösung mit Aktivkohle und gibt anschließend weitere Mineralsäure bis pH <2 hinzu.

### Beispiel

Ein Gemisch von 66 g II und 100 g Essigsäure wird 21 Std. gekocht. Man destilliert die Essigsäure ab, löst den Rückstand in 120 ml Ethanol, gibt 74 g 85%iges KOH und 75 ml Wasser hinzu und rührt 2 Std. bei 80°. Danach wird eingedampft. Man löst den Rückstand in 200 ml Wasser, gibt Salzsäure bis pH 7,5 hinzu, behandelt die Lösung mit Aktivkohle, filtriert und versetzt das Filtrat mit Salzsäure bis pH <2. Die ausgefallene freie Säure I wird abfiltriert, mit Wasser gewaschen und getrocknet. Ausbeute 45,4 g. F. 192-194° (aus Essigsäure).

## Patentansprüche

1. Verfahren zur Herstellung von 3-(3-Carboxypropyl)-5-methoxyindol-2-carbonsäure (I), dadurch gekennzeichnet, daß man 6-Carbethoxy-6-p-methoxyphenylhydrazonohexansäure (II) mit einer Carbonsäure, die 1-3 C-Atome enthält, cyclisiert, das erhaltene Gemisch aus I, den beiden I-Monoethylestern und I-Diethylester mit einer starken anorganischen Base behandelt und das erhaltene Salz durch Behandeln mit einer starken Säure in die freie Säure I umwandelt.

## Claims

1. Process for the preparation of 3-(3-carboxy-propyl)-5-methoxyindole-2-cabroxylic acid (I), characterized in that 6-carboethoxy-6-p-methoxyphenylhydrazono-hexanoic acid (II) is cyclized using a carboxylic acid which contains 1-3 carbon atoms, the resulting mixture comprising I, the two I monoethyl esters and I diethyl ester is treated with a strong inorganic base, and the resulting salt is converted into the free acid I by treatment with a stron acid.

## Revendications

1. Procédé pour préparer 3-(3-Carboxypropyl)-5-méthoxyindol-2-acide carboxylique (I), caractérisé en ce qu'on cyclise 6-Carbéthoxy-6-p-méthoxyphénylehydrazono-acide héxanique (II) avec un acide carboxylique de 1 à 3 atomes de carbone, que l'on traite le mélange de I, des deux monoéthylates de I et du diéthylate de I obtenu avec une forte base anorganique, et que l'on transforme le sel obtenu par traitement avec un acide fort en l'acide I libre.
